(19)

Europäisches Patentamt
European Patent Office
Office européen des brevets

(11) **EP 3 628 213 A1**

(12) **EUROPEAN PATENT APPLICATION**

(43) Date of publication:
**01.04.2020 Bulletin 2020/14**

(51) Int Cl.:
**A61B 5/00** *(2006.01)* **A61B 5/11** *(2006.01)*

(21) Application number: **18196574.0**

(22) Date of filing: **25.09.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **LONG, Xi**
  **5656 AE Eindhoven (NL)**
• **BEZEMER, Rick**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **DERIVING INFORMATION ABOUT A PERSON'S SLEEP AND WAKE STATES FROM A SEQUENCE OF VIDEO FRAMES**

(57)    For the purpose of deriving information about a person's sleep and wake states from a sequence of video frames, an arrangement (100) comprising a video camera (10) and a processing unit (20) is used. The video camera (10) serves for capturing a sequence of video frames during a time period, and the processing unit (20) is configured to process video frames provided by the video camera (10) and to provide output representative of the person's sleep and wake states during the time period. In particular, the processing unit (20) is configured to execute an algorithm according to which (i) a motion value-time relation, (ii) sets of features relating to respective epochs in the motion value-time relation and (iii) classifiers of the respective epochs are determined, wherein the algorithm is further configured to apply at least one of a personalized adaptive prior probability and a smoothing filter to the classifiers.

**Fig. 1**

**Description**

FIELD OF THE INVENTION

The invention relates to an arrangement designed to derive information about a person's sleep and wake states from a sequence of video frames, comprising a video camera for capturing a sequence of video frames during a time period, and a processing unit configured to process video frames provided by the video camera and to provide output representative of the person's sleep and wake states during the time period by executing an algorithm.

The invention further relates to a computer program product comprising a program code of a computer program to make a computer execute the algorithm of the arrangement as mentioned when the computer program is loaded on the computer.

BACKGROUND OF THE INVENTION

Sleep plays an important role in the development of neonates, both term and preterm infants. Monitoring of an infant's sleep is paramount for interpreting the infant's mental and physical development as well as sleep quality. Such monitoring is often based on an assessment of the wake-sleep pattern during a representative time period. For obtaining reliable information about the wake-sleep patterns, prolonged monitoring with multiple days is recommended.

Sensors attached to an infant's vulnerable skin would lead to irritation and damage to the skin. Therefore, it is desirable to use unobtrusive (noncontact) monitoring techniques. Many unobtrusive or minimal-obtrusive devices or systems have been developed and used for objective infant sleep monitoring. The functioning of such devices or systems is based on the use of various sensing techniques and various tools such as actigraphy, ballistocardiography, capacitive electrocardiogram, inductive sensors, photoplethysmography, and near infrared or thermal video camera. Especially camera-based baby monitors have been well commercialized in the market because of their advantage of total unobtrusiveness of monitoring.

In respect of the use of a video camera, it is noted that advanced algorithms of image processing for human tracking, which attempt to recognize an infant's face and/or body and the corresponding motion, are expected to work well in identifying the infant sleep states. However, such algorithms are computationally expensive since they need to deal with a large number of video frames, possibly requiring the use of a high-performance computing unit that is difficult and costly to integrate into the camera.

SUMMARY OF THE INVENTION

It is an object of the invention to provide a reliable way of monitoring a person's sleep and wake states without needing to apply expensive equipment. In this respect, it is noted that the fact that the invention is presented and explained in the context of sleep analysis of infants is not to be understood such as to imply that the invention is limited to such context. The fact is that the invention is equally applicable to monitoring sleep and wake states in older children and adults.

According to the invention, an arrangement is provided that is designed to derive information about a person's sleep and wake states from a sequence of video frames. The arrangement comprises a video camera for capturing a sequence of video frames during a time period, and a processing unit configured to process video frames provided by the video camera and to provide output representative of the person's sleep and wake states during the time period, wherein the processing unit is configured to execute an algorithm according to which (i) a motion value-time relation is determined from the video frames, (ii) sets of features relating to respective epochs in the motion value-time relation are determined by extracting a number of different features from the motion values in each of the respective epochs, and (iii) classifiers of the respective epochs are determined by classifying the respective sets of features relating to the respective epochs as being representative of the person's sleep or wake state. The algorithm is further configured to apply at least one of the following to the classifiers: (i) an adaptive prior probability determined for the particular person in dependence of the motion values of the respective epochs, and (ii) a smoothing filter removing short period deviations from an overall pattern of classifiers in respect of successive epochs.

When the invention is put to practice, a non-contact device in the form of a video camera is used to capture a video stream of a person that is assumed to be an infant in the following further explanation of the aspects of the invention. A processing unit is provided for executing an algorithm that is configured to quantify body motion from the video stream as a step in a process of reliable sleep/wake identification. Any suitable technique for determining the motion value-time relation from the video frames may be applied. For example, the algorithm may be configured to apply a technique known as 3D recursive search motion estimation so as to identify video actigraphy through time.

The video camera can be of any suitable type, and use can be made of an RGB camera or an infrared camera, for example. When it comes to the location of the camera with respect to an area where an infant can be present, such as a bed or an incubator, all that is required is that the camera is positioned such that it can have a view of at least a part of the infant's body. Apart from that, there is no specific requirement with respect to camera placement.

Sets of features representing body motion and possibly also relative possibility of being asleep is extracted from the motion value-time relation for respective epochs in the total time period (recording). It may be advantageous to normalize the features of each recording for the purpose of reducing between-infant and/or between-recording variability. The respective sets of features relating to the respective epochs are classified as being representative of the person's sleep or wake state so that a classifier is determined for each of the respective epochs. For example, the algorithm may be configured to apply Bayesian-based linear discriminant analysis for determining the classifiers of the respective epochs, which does not alter the fact that within the framework of the invention, other types of analysis may be applied as well.

Putting the invention to practice may further involve performing a personalized method to adapt the classifier's prior probability for each recording, in order to find the prior probability for each particular person in each particular recording. Also, a filter such as a median filter may be used to smoothen sleep and wake classification results, and to generate final classification results. Information about the way in which the classifiers are further adapted may be used in machine learning.

In respect of the classifier's prior probability, it is noted that any information as can be derived from a distribution of the motion values over the respective epochs and/or a total percentage of motions over an entire recording may be used for determining the priors. For example, the algorithm may be configured to determine the adaptive prior probability in dependence of a number value of epochs with non-zero motion values or motion values which are larger than a certain minimum, *i.e.* a number value of epochs with motion values which are larger than a reference motion value that is equal to or higher than zero. In that respect, the algorithm may particularly be configured to assign a high prior probability for wake when the number value of epochs with motion values which are larger than the reference motion value is equal to or higher than a threshold, which may be an experimentally chosen threshold, and to assign a low prior probability for wake when the number value of epochs with motion values which are larger than the reference motion value is lower than the threshold. Alternatively, the algorithm may be configured to determine an optimal relation between the number value of epochs with motion values which are larger than the reference motion value and a prior probability for wake. Examples of the number value of the epochs with motion values which are larger than the reference motion value include a percentage or other statistics such as mean, median, standard deviation, inter-quartile range and entropy. A practical example of an optimal relation includes an optimal linear relation as may be determined by applying linear regression. Other examples include per-defined correlation coefficient, non-linear relation and piecewise linear/non-linear relation.

In order to have the filter as mentioned, the algorithm may be configured to assess whether two sequences of epochs both representing a minimum length of time and involving sets of features to be classified as being representative of only one of the person's sleep or wake state are interrupted by one epoch or a limited number of epochs involving (a) set(s) of features to be classified as being representative of the other of the person's sleep or wake state, and if such is found to be the case, to set the classifier(s) of the one epoch or the limited number of epochs to be the same as the classifiers of the two sequences of epochs. In that way, the results smoothing effect is obtained, wherein classifiers suggesting a short wake state are removed from long sleep periods, and wherein classifiers suggesting a short sleep state are removed from long wake periods. For example, a number of epochs not higher than 2 or 3 may be regarded as a limited number of epochs.

The features to be extracted from the motion values in each of the respective epochs may be any suitable type of features, such as features which represent a motion aspect. For example, the features include at least one of (i) the mean of the motion values in each of the respective epochs and (ii) the number of non-zero motion values or motion values which are larger than a certain minimum, *i.e.* motion values which are larger than a reference motion value that is equal to or higher than zero, in each of the respective epochs. Additionally, the features to be extracted from the motion values in each of the respective epochs may include a relative possibility of sleep, wherein the algorithm may be configured to include a step of determining a time distance of each of the respective epochs to a nearest epoch with a high activity level in a process of determining the relative possibility of sleep. In this respect, it is noted that, relying on at least one of the mean of the motion values om each of the respective epochs and the number of motion values which are larger than the reference motion value in each of the respective epochs, the features to be extracted from the motion values in each of the respective epochs may further include at least one of (i) a relative possibility of sleep, wherein the algorithm is configured to include a step of determining a time distance of each of the respective epochs to a nearest epoch with a high activity level in a process of determining the relative possibility of sleep, and wherein the algorithm is configured to identify the epochs with a high activity level by taking the epochs with the highest mean of the motion values, up to a predetermined maximum percentage of a total number of epochs, and (ii) a relative possibility of sleep, wherein the algorithm is configured to include a step of determining a time distance of each of the respective epochs to a nearest epoch with a high activity level in a process of determining the relative possibility of sleep, and wherein the algorithm is configured to identify the epochs with a high activity level by taking the epochs with the highest number of motion values which are larger than the reference motion value, up to a predetermined maximum percentage of a total number of epochs. It is understood that involving the relative possibility of sleep in a process of identifying sleep/wake states contributes to the accuracy of the final output of the process.

As mentioned earlier, the algorithm may be configured to normalize the features, so that the influence of variations, such as variations between infants and/or variations between recordings may be reduced. Any normalization process may be used, including Z-score, max-min, quantile, percentile, and unit standard deviation.

Advantageously, the processing unit is designed for machine learning so that analysis results may even be further improved, *i.e.* may provide an even better detection of actual situations, during the lifetime of the arrangement. For example, the algorithm may be configured to determine machine learning classifiers on the basis of differences between (i) an initial set of classifiers determined on the basis of the features and (ii) a final set of classifiers determined by applying at least one of the adaptive prior probability and the smoothing filter, and to use the machine learning classifiers for making adjustments in the algorithm as far as determining the classifiers of the respective epochs is concerned.

Especially in the context of infant care, it may be desirable to take into account the fact that motion may result from an infant being subjected to a caretaking action. In view thereof, an arrangement of the invention that is particularly designed for use in infant care is feasible, and that involves a special algorithm that is configured to classify the respective sets of features relating to the respective epochs as being representative of a care state of the infant besides the infant's sleep or wake state. In that case, the algorithm may be configured to set the classifier of an epoch to be a care state classifier when the epoch is an epoch with an activity level that is above a threshold chosen to distinguish between the wake state and the care state.

In a practical embodiment of the invention, a computer program product may be provided, comprising a program code of a computer program to make a computer execute the algorithm when the computer program is loaded on the computer.

The above-described and other aspects of the invention will be apparent from and elucidated with reference to the following detailed description of items of the theoretical background of the invention and actual ways of putting the invention to practice.

BRIEF DESCRIPTION OF THE DRAWINGS

The invention will now be explained in greater detail with reference to the figures, in which equal or similar parts are indicated by the same reference signs, and in which:

Fig. 1 diagrammatically shows a video camera and a processing unit of the arrangement according to the invention, as used for monitoring sleep and wake states of an infant in an incubator,
Fig. 2 is a diagram of various steps of an algorithm to be executed by the processing unit for the purpose of providing output representative of the infant's sleep and wake states during a recording,
Fig. 3 shows a first example of a graph of a motion value-time relation and monitoring results associated therewith,
Fig. 4 shows a second example of a graph of a motion value-time relation and monitoring results associated therewith, and
Fig. 5 shows a scatter plot and a linear fitting between percentage of epochs with non-zero motion values and percentage of wake of a number of recordings.

DETAILED DESCRIPTION OF EMBODIMENTS

As explained in the foregoing, the invention is about obtaining reliable information about a person's sleep in a non-obtrusive way, particularly by capturing a sequence of video frames and using a processing unit that is programmed to follow a certain algorithm in analyzing the video frames. In many cases, when a certain time period is considered, it is desirable to know during which epochs of the time period the person was asleep and during which epochs the person was awake. According to the invention, video detection of motion of the person is at the basis of the analysis to be performed for gaining the knowledge as desired.

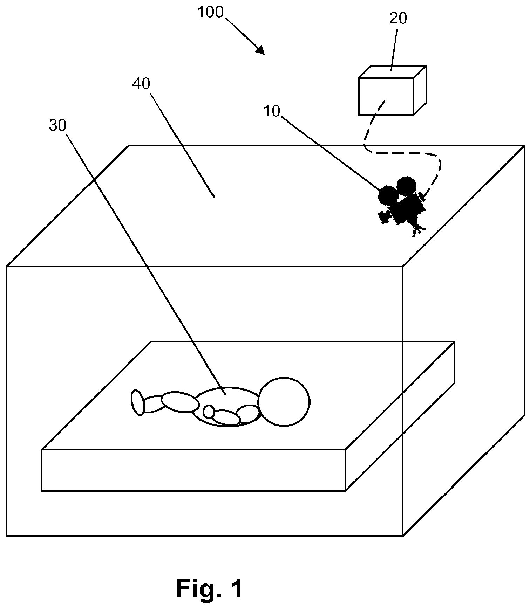

Fig. 1 diagrammatically shows a video camera 10 and a processing unit 20 of an arrangement 100 according to the invention, as used for monitoring sleep and wake states of an infant 30 in an incubator 40. The positioning of the video camera 10 with respect to the incubator 40 is not very critical, as in fact the only requirement is to have the video camera 10 arranged at a position where the video camera 10 is capable of recording motion of at least some part of the infant's body. In order to keep costs of the arrangement 100 as low as possible, it is advantageous to use only one video camera 10, but that does not alter the fact that the invention covers the use of two or even more video cameras 10 as well.

The processing unit 20 can be provided in various ways. Practical examples include an arrangement of the processing unit 20 as an integral part of the video camera 10 and an arrangement of the processing unit 20 in a computer system positioned separately from the video camera 10. In any case, the processing unit 20 is arranged and configured so as to be enabled to receive information from the video camera 10. Further, any type of display device such as a screen (not shown) can be used for displaying information output from the processing unit 20 to a user. Communication between one or more components of the arrangement 100 for the purpose of conveying information can take place through a wired system or in a wireless manner, wherein use can be made of the Internet if so desired.

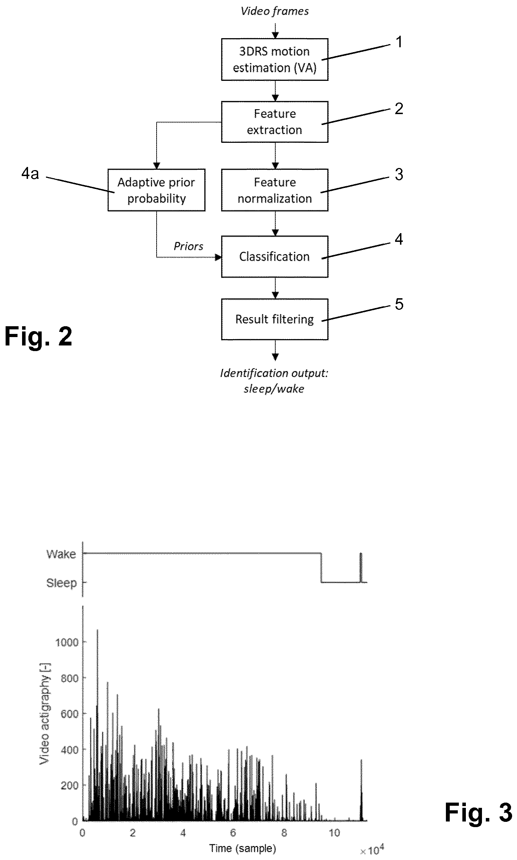

Fig. 2 is a diagram of various steps of the algorithm to be executed by the processing unit 20 for the purpose of providing output representative of the infant's sleep and wake states during a recording. The following description refers to the various steps and provides further information about each of those steps, wherein it is assumed that the invention is applied in a context as illustrated in Fig. 1, *i.e.* a context in which it is desirable to obtain information about a wake-sleep pattern of an infant 30 in an incubator 40. However, it is understood that the invention is also applicable to other contexts, including a domestic context in which an infant (or an older person) can be monitored while lying in bed.

Motion or video actigraphy, *i.e.* motion estimated from a video recording, is mainly caused by body motion, parent activity/caretaking, or other disturbances, e.g. from other moving objects. This is important information that can tell whether the infant 30 is present in the incubator 40, enabling further an automated analysis of the infant's sleep. The main idea is that body motion of the infant 30 is highly associated with the infant's sleep and wake states, assuming that an infant 30 usually has a higher degree of body motion during the wake state compared with the degree of body motion during the sleep state.

In a first step 1 of the analysis of the video frames, a motion estimation technique is employed for quantifying motion as video actigraphy (VA). In the shown example, the motion estimation technique is assumed to be a technique known as 3D recursive search (3DRS). It has been demonstrated that this particular technique is robust to scene changes, especially light changes, meaning that the effect of daytime light can be eliminated. The type of video recording may be RGB at grayscale. Another feasible example of the type of video recording is NIR, wherein it is noted that the application of RGB may be best applicable to the context of preterm infants, while the application of NIR may be best applicable to the context of term infants. The raw 3DRS motion estimates corresponding to the video recording may have a frame rate of approximately 15 Hz, but may also have another frame rate such as 8 Hz, depending on the video camera 10 that is employed.

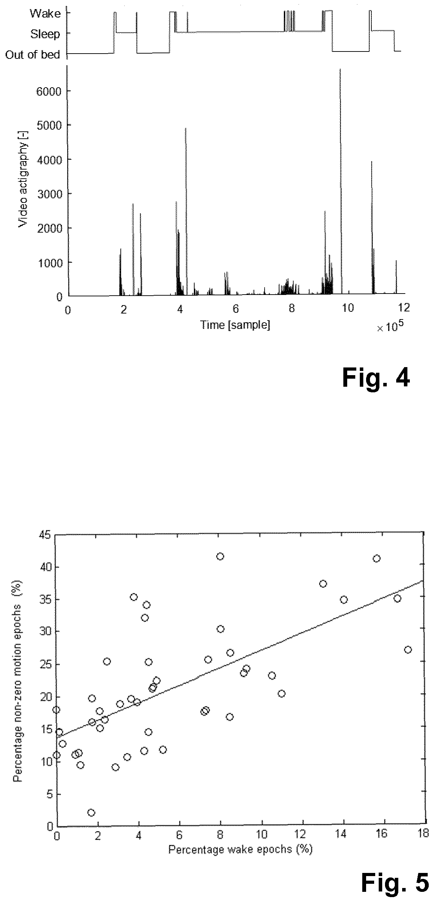

Fig. 3 shows an example of VA values relating to a preterm infant 30, obtained by running a 3DRS algorithm for processing video frames of a recording of about 2 hours taken at a frequency of about 15 Hz. Fig. 4 shows an example of VA values relating to a healthy term infant 30, obtained by running a 3DRS algorithm for processing video frames of a recording of about 24 hours taken at the same frequency of about 15 Hz. A larger estimated VA value usually corresponds to a large body movement or more body movements.

In a second step 2 of the analysis of the video frames, features are extracted from the raw estimated motion data. According to guidelines of the American Academy of Sleep Medicine (AASM), sleep states should be classified for continuous non-overlapping epochs of 30 seconds. In view thereof, it is assumed that the features are extracted on a 30 seconds basis. In the following, it is explained how four features can be extracted for each epoch, two of those features being computed based on the mean of the VA values over the respective epochs, and another two of those features being computed by counting the non-zero VA values over the respective epochs. The set of four features that is obtained in this way for each of the epochs is summarized as follows:

(i) video actigraphy mean (VAM) = mean of VA values (motion estimates) over 30 seconds, computed based on raw VA data after 3DRS motion estimations,
(ii) video actigraphy count (VAC) = (averaged) count of non-zero VA values (motion estimates) over 30 seconds, computed based on raw VA data after 3DRS motion estimations,
(iii) relative possibility of sleep based on VAM ($PS_{VAM}$) = relative possibility of sleep, as measured by the time distance to the nearest epoch with a high activity level as computed based on VAM, and
(iv) relative possibility of sleep based on VAC ($PS_{VAC}$) = relative possibility of sleep, as measured by the time distance to the nearest epoch with a high activity level as computed based on VAC.

In the following, further details of the various features and the way in which they are determined are provided.
The VAM aims to capture the average magnitude of body movements, while the VAC characterizes movement frequency, *i.e.* number of movements, for each epoch. Assuming the raw VA data within a 30 seconds epoch is u = {$u_1$, $u_2$, ..., $u_k$}, where k is 450 when the frequency of the video frames is 15 Hz, the mean of the VA data over the epoch is computed by

$$VAM \; = \; \frac{\sum_{i=1}^{k} u_i}{k}$$

and the (averaged) count of non-zero VA data over the epoch is computed by

$$VAC = \frac{\sum_{i=1}^{k} v_i}{k}$$

where

$$v_i = \begin{cases} 1, & u_i > 0 \\ 0, & Otherwise, \end{cases} \quad for\ all\ i\ (i = 1, 2, ..., k)$$

It is challenging to identify wakefulness in situations of reduced body movements, e.g. during quiet wake, with VAM or VAC exclusively. Further accuracy can be realized by extracting features to characterize the possibility of being asleep $PS_{VAM}$ and $PS_{VAC}$ before/after very high activity level. This can be done by quantifying the logarithm of time difference between each epoch and its nearest epoch with a lot of body movements, in correspondence to a large VAM value or a large VAC value. The outcome can be smoothed through a moving average operation with an experimentally optimized window of 10 minutes. For each recording, an epoch with a VAM or VAC value higher than the 95 percentile of the VAM or VAC values over the entire recording is considered to have a high activity level for the purpose of computing $PS_{VAM}$ or $PS_{VAC}$ values. The hypothesis is that epochs closer to an epoch with a high level of activity, and thereby with a smaller time difference, are more likely to correspond to wake state, albeit possibly with less body movements. Assuming a series of n epoch-based VAM or VAC feature values a = {$a_1$, $a_2$, ..., $a_n$} over an entire recording, $a_T$ is a subset of a in which feature values are larger than a threshold T, where the associated epoch indices are $e_T$ = {$e_1$, $e_2$, ..., $e_m$}. Accordingly, b = {$b_1$, $b_2$, ..., $b_n$} is a set of $PS_{VAM}$ or $PS_{VAC}$ feature values from the same series. The value $b_x$ at epoch x (x = 1, 2, ..., n) can then be computed such that

$$b_x = \ln(\min\{|x - e_1|, |x - e_2|, ..., |x - e_m|\})$$

T may be experimentally chosen as the 95 percentile of the feature values over the entire recording, for example. In any case, it follows from the foregoing that the second step 2 of the analysis of the video frames may involve obtaining two new, secondary features $PS_{VAM}$ and $PS_{VAC}$ after computing on the two primary features VAM and VAC.

In a third step 3 of the analysis of the video frames, in order to reduce the global variability between subjects and between days conveyed by VA, features are normalized for each recording. For $PS_{VAM}$ and $PS_{VAC}$, feature values may be normalized to zero mean and unit standard deviation of the entire recording (Z-score normalization), while for VAM and VAC, it may be practical to normalize the feature values between 0 and 1 (max-min normalization). Within the framework of the invention, any suitable type of normalization method may be applied, wherein it is noted that normalization methods can be different for different features.

In a fourth step 4 of the analysis of the video frames, classification takes place on the basis of the sets of features of the respective epochs. In particular, for each epoch, the set of features of that particular epoch is classified as being representative of a wake state or a sleep state, possibly also an out of bed state. In Figs. 3 and 4, the outcome of the classification process is represented above the respective graphs. The classifiers of the respective epochs can be determined in any suitable manner. For example, it may be practical to use Bayesian-based linear discriminant analysis in the process.

The processing unit 20 is configured to take the prior probability of the classifiers into account. This step of the analysis of the video frames is a parallel step that is indicated by reference numeral 4a in Fig. 2. In general, the prior probability of a classifier is usually set to give a preference to one class when making decisions. On the basis of the assumption that body movements indicate a wake state, it is intuitive that a recording having more epochs with body movements should have more wake epochs, leading to a higher probability for wake epochs and a lower probability for sleep epochs. This is confirmed by the significant correlation shown in Fig. 5 in which a relation between a percentage of non-zero motion epochs and a percentage of wake epochs is illustrated. Therefore, the invention proposes a personalized adaptive priors depending on the percentage of non-zero motions, which can be computed based on VAM or VAC, for example. When it is assumed that the prior probability is PriW for wake and 1-PriW for sleep, and that the percentage of epochs with non-zero motions is PE, PE is compared to a threshold S. If it appears that PE is larger than S, a higher PriW (PriW_high) should be assigned, otherwise, a lower PriW (PriW low) should be given, such that

$$PriW = \begin{cases} PriW\_low, & PE < S \\ PriW\_high, & PE \geq S \end{cases}$$

In an example involving experimentally chosen values, S is 0.14, the higher PriW is 0.5, and the lower PriW is 0.1. The personalized prior probability can also be determined using a linear regression method, wherein an optimal linear relation between PE and PriW is established.

Fig. 5 shows a scatter plot and illustrates a linear fitting between the percentage of non-zero motion epochs and the percentage of wake epochs. The scatter plot is derived from 45 recordings for healthy term infants. Pearson's correlation coefficient is 0.66 at $p < 0.0001$.

In a fifth step 5 of the analysis of the video frames, suspicious segmented awakenings or sleep states with a very short time duration are "filtered" out, while relatively long periods of wake and sleep are preserved to be annotated. It is therefore advantageous to apply a "low pass filter" to smoothen the detection results, as by doing so, it may be possible to correct some single or very short periods of misclassified wake and sleep epochs. The window size can be experimentally chosen to optimize classification performance.

Finally, the processing unit 20 outputs the identification output that can be communicated to and interpreted by a user in order for the user to be provided with knowledge about the wake-sleep behavior of a recorded infant 30 over the time of the recording.

In the context of the invention, experiments were performed to validate the above-described algorithm, and to check whether the invention is suitable to be used for obtaining reliable information about the wake-sleep behavior of both healthy term infants and preterm infants. For preterm infants, 45 video recordings (738x480 pixels or 768x576 pixels) from 7 infants with an average gestational age of 29.9 weeks were included. For healthy term infants, video data of 29 hours (1280x720 pixels) from 8 infants aged 6 months on average were included. Inclusion criteria were that the term infants needed to sleep (most of the time) in their own bed and bedroom. Sleep and wake states were scored by human annotators for non-overlapping epochs that lasted 30 seconds. These annotations served as the golden standard for automated classification. For preterm infants, caretaking was also annotated and considered as wake, yet there were much less wake epochs.

To demonstrate the validity of the proposed classification algorithm, a (subject-independent) leave-one-subject-out cross validation (LOOCV) was applied. Overall accuracy and the chance-compensated metric Cohen's Kappa coefficient were used to assess the classification performance. The following tables present and compare the sleep and wake classification results using different feature sets and settings/methods for preterm infants and healthy term infants, respectively. It can be seen that using adaptive prior probability for the individual infants and result filtering can improve the classification performance for both preterm and term infants.

| Sleep and wake (and caretaking) classification results for preterm infants (average and standard deviation over infants, LOOCV) | | | |
|---|---|---|---|
| Feature set | Setting | Accuracy | Cohen's Kappa |
| VAM+PS$_{VAM}$ | Basic classifier<br>+ result filtering | 0.82 ± 0.11<br>0.85 ± 0.11 | 0.38 ± 0.14<br>0.42 ± 0.16 |
| VAC+PS$_{VAC}$ | Basic classifier<br>+ result filtering | 0.79 ± 0.16<br>0.81 ± 0.17 | 0.39 ± 0.16<br>0.41 ± 0.15 |
| Best set (on Kappa):<br>VAM+VAC+PS$_{VAC}$ | Basic classifier<br>+ result filtering | 0.81 ± 0.15<br>0.83 ± 0.15 | 0.40 ± 0.17<br>0.44 ± 0.18 |
| Note: Adaptive prior probability is not available/applicable due to short recordings from preterm infants. Median filter window size for result filtering was chosen to optimize Kappa. | | | |

| Sleep and wake (and caretaking) classification results for healthy term infants (average and standard deviation over infants, LOOCV) | | | |
|---|---|---|---|
| Feature set | Setting | Accuracy | Cohen's Kappa |
| VAM+PS$_{VAM}$ | Basic classifier<br>+ adaptive prior probability +<br>result filtering | 0.85 ± 0.10<br>0.89 ± 0.04 | 0.48 ± 0.15<br>0.55 ± 0.08 |
| VAC+PS$_{VAC}$ | Basic classifier<br>+ adaptive prior probability +<br>result filtering | 0.90 ± 0.03<br>0.92 ± 0.02 | 0.52 ± 0.14<br>0.59 ± 0.16 |

(continued)

| Sleep and wake (and caretaking) classification results for healthy term infants (average and standard deviation over infants, LOOCV) | | | |
|---|---|---|---|
| Feature set | Setting | Accuracy | Cohen's Kappa |
| Best set (on Kappa): VAM+VAC+PS$_{VAC}$ | Basic classifier + adaptive prior probability + result filtering | 0.90 ± 0.03 0.93 ± 0.02 | 0.53 ± 0.15 0.60 ± 0.16 |
| Note: Median filter window size for result filtering was chosen to optimize Kappa. | | | |

The sleep and wake classification results can be used for higher level interpretations, e.g. total time of infant in bed, total sleep time, total wake time, number of awakenings, and other infant sleep/wake statistics. As mentioned earlier, the invention also covers application of the proposed algorithm for the purpose of monitoring wake-sleep behavior of persons older than infants. The arrangement 100 according to the invention can be used in homes, hospitals and other settings. Various reasons for applying the invention are available, including a desire to obtain sleep quality information of a person and a desire to schedule caretaking actions during wake states as much as possible.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details that are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The term "comprise" as used in this text will be understood by a person skilled in the art as covering the term "consist of'. Hence, the term "comprise" may in respect of an embodiment mean "consist of', but may in another embodiment mean "contain/include at least the defined species and optionally one or more other species".

A possible summary of the invention reads as follows. For the purpose of deriving information about a person's sleep and wake states from a sequence of video frames, an arrangement comprising a video camera 10 and a processing unit 20 is used. The video camera 10 serves for capturing a sequence of video frames during a time period, and the processing unit 20 is configured to process video frames provided by the video camera 10 and to provide output representative of the person's sleep and wake states during the time period. In particular, the processing unit 20 is configured to execute an algorithm according to which (i) a motion value-time relation, (ii) sets of features relating to respective epochs in the motion value-time relation and (iii) classifiers of the respective epochs are determined, wherein the algorithm is further configured to apply at least one of (i) a personalized adaptive prior probability, *i.e.* an adaptive prior probability determined for the particular person in dependence of the motion values of the respective epochs, and (ii) a smoothing filter to the classifiers.

**Claims**

1. Arrangement (100) designed to derive information about a person's sleep and wake states from a sequence of video frames, comprising

   - a video camera (10) for capturing a sequence of video frames during a time period, and
   - a processing unit (20) configured to process video frames provided by the video camera (10) and to provide output representative of the person's sleep and wake states during the time period, wherein the processing unit (20) is configured to execute an algorithm according to which

- a motion value-time relation is determined from the video frames,
- sets of features relating to respective epochs in the motion value-time relation are determined by extracting a number of different features from the motion values in each of the respective epochs, and
- classifiers of the respective epochs are determined by classifying the respective sets of features relating to the respective epochs as being representative of the person's sleep or wake state,
- wherein the algorithm is further configured to apply at least one of the following to the classifiers:

   - an adaptive prior probability determined for the particular person in dependence of the motion values of the respective epochs, and
   - a smoothing filter removing short period deviations from an overall pattern of classifiers in respect of successive epochs.

2. Arrangement (100) according to claim 1, wherein the algorithm is configured to determine the adaptive prior probability in dependence of a distribution of the motion values over the respective epochs.

3. Arrangement (100) according to claim 2, wherein the algorithm is configured to determine the adaptive prior probability in dependence of a number value of epochs with motion values which are larger than a reference motion value that is equal to or higher than zero in a total of epochs.

4. Arrangement (100) according to claim 3, wherein the algorithm is configured to assign a high prior probability for wake (PriW high) when the number value of epochs with motion values which are larger than the reference motion value is equal to or higher than a threshold (S), and to assign a low prior probability for wake (PriW low) when the number value of epochs with motion values which are larger than the reference motion value is lower than the threshold (S).

5. Arrangement (100) according to claim 3, wherein the algorithm is configured to determine an optimal relation between the number value of epochs with motion values which are larger than the reference motion value and a prior probability for wake.

6. Arrangement (100) according to any of claims 1-5, wherein, in order to have the smoothing filter, the algorithm is configured to assess whether two sequences of epochs both representing a minimum length of time and involving sets of features to be classified as being representative of only one of the person's sleep or wake state are interrupted by one epoch or a limited number of epochs involving (a) set(s) of features to be classified as being representative of the other of the person's sleep or wake state, and if such is found to be the case, to set the classifier(s) of the one epoch or the limited number of epochs to be the same as the classifiers of the two sequences of epochs.

7. Arrangement (100) according to any of claims 1-6, wherein the features to be extracted from the motion values in each of the respective epochs include at least one of (i) the mean of the motion values in each of the respective epochs and (ii) the number of motion values which are larger than a reference motion value that is equal to or higher than zero in each of the respective epochs.

8. Arrangement (100) according to claim 7, wherein the features to be extracted from the motion values in each of the respective epochs include a relative possibility of sleep, and wherein the algorithm is configured to include a step of determining a time distance of each of the respective epochs to a nearest epoch with a high activity level in a process of determining the relative possibility of sleep.

9. Arrangement (100) according to claim 7, wherein the features to be extracted from the motion values in each of the respective epochs include at least one of (i) a relative possibility of sleep, wherein the algorithm is configured to include a step of determining a time distance of each of the respective epochs to a nearest epoch with a high activity level in a process of determining the relative possibility of sleep, and wherein the algorithm is configured to identify the epochs with a high activity level by taking the epochs with the highest mean of the motion values, up to a predetermined maximum percentage of a total number of epochs, and (ii) a relative possibility of sleep, wherein the algorithm is configured to include a step of determining a time distance of each of the respective epochs to a nearest epoch with a high activity level in a process of determining the relative possibility of sleep, and wherein the algorithm is configured to identify the epochs with a high activity level by taking the epochs with the highest number of motion values which are larger than the reference motion value, up to a predetermined maximum percentage of a total number of epochs.

10. Arrangement (100) according to any of claims 1-9, wherein the algorithm is configured to normalize the features.

11. Arrangement (100) according to any of claims 1-10, wherein the algorithm is configured to determine machine learning classifiers on the basis of differences between (i) an initial set of classifiers determined on the basis of the features and (ii) a final set of classifiers determined by applying at least one of the adaptive prior probability and the smoothing filter, and to use the machine learning classifiers for making adjustments in the algorithm as far as determining the classifiers of the respective epochs is concerned.

12. Arrangement (100) according to any of claims 1-11, wherein the algorithm is configured to apply 3D recursive search motion estimation for determining the motion value-time relation from the video frames.

13. Arrangement (100) according to any of claims 1-12, wherein the algorithm is configured to apply Bayesian-based linear discriminant analysis for determining the classifiers of the respective epochs.

14. Arrangement (100) according to any of claims 1-13, designed for use in infant care, wherein the algorithm is configured to classify the respective sets of features relating to the respective epochs as being representative of a care state of the infant (30) besides the infant's sleep or wake state, and wherein the algorithm is configured to set the classifier of an epoch to be a care state classifier when the epoch is an epoch with an activity level that is above a threshold chosen to distinguish between the wake state and the care state.

15. Computer program product comprising a program code of a computer program to make a computer execute the algorithm of the arrangement (100) according to any of claims 1-14 when the computer program is loaded on the computer.

**Fig. 1**

Video frames

↓

3DRS motion
estimation (VA) — 1

↓

Feature
extraction — 2

↓

Adaptive prior
probability 4a

Feature
normalization — 3

↓

Priors → Classification — 4

↓

Result filtering — 5

↓

Identification output:
sleep/wake

# Fig. 2

Fig. 3

**Fig. 4**

**Fig. 5**

Europäisches
Patentamt

European
Patent Office

Office européen
des brevets

# EUROPEAN SEARCH REPORT

Application Number

EP 18 19 6574

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/364617 A1 (SILBERSCHATZ PAUL JOSEPH [US] ET AL) 15 December 2016 (2016-12-15) * paragraphs [0040] - [0042], [0056] - [0070] * | 1-15 | INV. A61B5/00 A61B5/11 |
| A | GUNNARSDOTTIR KRISTIN M ET AL: "A Novel Sleep Stage Scoring System: Combining Expert-Based Rules with a Decision Tree Classifier", 2018 40TH ANNUAL INTERNATIONAL CONFERENCE OF THE IEEE ENGINEERING IN MEDICINE AND BIOLOGY SOCIETY (EMBC), IEEE, 18 July 2018 (2018-07-18), pages 3240-3243, XP033429087, DOI: 10.1109/EMBC.2018.8513039 [retrieved on 2018-10-26] * the whole document * | 1-15 | |
| A | JP 2012 000375 A (RITSUMEIKAN) 5 January 2012 (2012-01-05) * paragraphs [0036], [0037], [0044] * | 1-15 | TECHNICAL FIELDS SEARCHED (IPC) A61B |
| A | WO 2016/193030 A1 (KONINKLIJKE PHILIPS NV [NL]) 8 December 2016 (2016-12-08) * page 9, line 25 - page 10, line 11 * | 12 | |
| A | WO 2017/196695 A2 (UDISENSE INC [US]) 16 November 2017 (2017-11-16) * page 5, lines 18-25 * * page 11, lines 13-29 * * claim 14 * | 14 | |

The present search report has been drawn up for all claims

| Place of search | Date of completion of the search | Examiner |
|---|---|---|
| Berlin | 15 March 2019 | Pohjamo, Terhi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 19 6574

This annex lists the patent family members relating to the patent documents cited in the above-mentioned European search report.
The members are as contained in the European Patent Office EDP file on
The European Patent Office is in no way liable for these particulars which are merely given for the purpose of information.

15-03-2019

| Patent document cited in search report | | Publication date | Patent family member(s) | | Publication date |
|---|---|---|---|---|---|
| US 2016364617 | A1 | 15-12-2016 | US 2016364617 A1<br>US 2019065862 A1<br>WO 2016205246 A1 | | 15-12-2016<br>28-02-2019<br>22-12-2016 |
| JP 2012000375 | A | 05-01-2012 | NONE | | |
| WO 2016193030 | A1 | 08-12-2016 | NONE | | |
| WO 2017196695 | A2 | 16-11-2017 | CN 109074717 A<br>EP 3455839 A2<br>WO 2017196695 A2 | | 21-12-2018<br>20-03-2019<br>16-11-2017 |

EPO FORM P0459

For more details about this annex : see Official Journal of the European Patent Office, No. 12/82